# EUROPEAN PATENT SPECIFICATION

(11) **EP 3 840 649 B1**
(45) Date of publication and mention of the grant of the patent: **28.06.2023**
(21) Application number: 19759124.1
(22) Date of filing: 06.08.2019
(51) Int. Cl.: A61B 5/15, B01L 3/00, A61M 39/10, A61M 25/00, A61M 25/06

(54) **BLOOD COLLECTION DEVICES**
BLUTSAMMELVORRICHTUNGEN
DISPOSITIFS DE COLLECTE DE SANG

(30) Priority: 23.08.2018 US 201862722058 P; 05.08.2019 US 201916532178
(43) Date of publication of application: 30.06.2021
(73) Proprietor: Becton, Dickinson and Company, Franklin Lakes, NJ 07417 (US)
(72) Inventor: KUNARDI, Linda, Serangoon 550305 (SG)
(74) Representative: dompatent von Kreisler Selting Werner - Partnerschaft von Patent- und Rechtsanwälten mbB
(86) International application number: PCT/US2019/045328
(87) International publication number: WO 2020/040987

(56) References cited:
- EP-A1- 2 380 665
- EP-A1- 3 042 691
- WO-A1-2011/073729
- WO-A2-2011/071897
- US-A1- 2006 037 903
- US-A1- 2017 014 310
- US-B1- 7 387 216

## Description

### BACKGROUND

A VACUTAINER^{™} blood collection tube may include a sterile glass or plastic test tube with a rubber stopper that creates a vacuum inside of the tube, facilitating the drawing of a predetermined volume of blood from a patient. In order to draw blood from the patient, a double-ended needle may be used. A proximal end of the needle may be positioned inside a holder, and a distal end of the needle may be inserted into a vein of a patient. When the VACUTAINER^{™} blood collection tube is inserted into the holder, the rubber stopper may be punctured by the proximal end of the needle, and the vacuum in the tube may pull blood through the needle into the tube. The filled tube may then be removed and another tube can be inserted and filled the same way.

Related Technologies are provided in US 2017/014310 A1, EP 3 042 691 A1, WO 2011/071897 A2, EP 2 380 665 A1, US 7 387 216 B1, US 2006/037903 A1 and WO 2011/073729 A1.

US 2017/014310 A1 discloses a blood collection apparatus comprising: a connector comprising a distal end, a proximal end, and a lumen extending there between, wherein the distal end comprises a male luer lock fitting configured to couple to a proximal end of a catheter adapter, and wherein the proximal end comprises a male luer slip fitting.

EP 2 380 665 A1 discloses a blood collection apparatus comprising: a lid for a blood collection tube, the lid comprising: a body having a cover portion and an inner wall extending from the cover portion, the inner wall aligned with an aperture extending through the cover portion, the inner wall and the aperture forming a channel; a closure; and a tether connecting the body to the closure, wherein the closure comprises an annular flange that fits inside and contacts the circumference of the channel when the closure is in a closed position.

This method of drawing blood can be expensive due to the cost of the needle, which is metal, as well as the rubber stopper. Furthermore, the needle may pose a hazard to clinicians. Clinicians in some markets, due to cost and other factors, may not use the VACUTAINER^{™} approach, and may resort to more dangerous practices that increase a risk of blood exposure. There is a need for blood collection systems and methods that are safe and cost-effective.

The subject matter claimed herein is not limited to embodiments that solve any disadvantages or that operate only in environments such as those described above. Rather, this background is only provided to illustrate one example technology area where some implementations described herein may be practiced.

### SUMMARY

The present disclosure relates generally to a blood collection apparatus and related methods. The invention is set out in the appended set of claims. A method is not claimed.

In some embodiments, the blood collection apparatus may include a connector. In some embodiments, the connector may include a distal end, a proximal end, and a lumen extending there between. In some embodiments, the distal end may include a male luer lock fitting configured to couple to a proximal end of a catheter adapter, which may include a female luer fitting. In some embodiments, the proximal end of the connector may include a male luer slip fitting.

In some embodiments, the blood collection apparatus may include a lid for a blood collection tube. In some embodiments, the lid may include a body, which may include a cover portion and an inner wall extending from the cover portion. In some embodiments, the inner wall may be aligned with an aperture extending through the cover portion. In some embodiments, the inner wall and the aperture may form a channel configured to receive the male luer slip fitting of the connector. In some embodiments, the inner wall may be tapered to receive the male luer slip fitting of the connector.

In some embodiments, the lid may include a closure and/or a tether connecting the body to the closure. In some embodiments, the closure may include an annular flange, which may fit inside and contact a circumference of the channel when the closure is in a closed position. In some embodiments, the annular flange may include an outer circumference less than a circumference of the channel. In some embodiments, the annular flange may include an outer circumference larger than a circumference of the channel or approximately a same size as the circumference of the channel.

In some embodiments, the body of the lid may include a generally cylindrical outer wall. In some embodiments, the closure may include another annular flange, which may contact the outer circumference of the outer wall when the closure is in a closed position. In some embodiments, the other annular flange may include an inner circumference greater than the outer circumference of the outer wall. In some embodiments, the other annular flange may include an inner circumference less than or approximately equal to the outer circumference of the outer wall.

In some embodiments, an outer surface of the cover portion may be generally planar. In some embodiments, an inner surface of the cover portion disposed between the inner wall and the outer wall is generally planar.

In some embodiments, the blood collection apparatus may include the blood collection tube coupled to the lid. In some embodiments, the blood collection tube may include a rim forming an opening into a cavity of the blood collection tube. In some embodiments, the inner wall may extend through the opening. In some embodiments, the rim may contact an inner surface of the outer wall of the body. In some embodiments, the inner surface of the outer wall may include one or more grooves, which may extend generally parallel to a central axis of the lid. In some embodiments, the grooves may be permeable to air but not blood.

### BRIEF DESCRIPTION OF THE SEVERAL VIEWS OF THE DRAWINGS

Example embodiments will be described and explained with additional specificity and detail through the use of the accompanying drawings in which:
Figure 1A is an upper perspective view of an example lid, according to some embodiments;
Figure 1B is a lower perspective view of the lid of Figure 1A, according to some embodiments;
Figure 1C is a cross-sectional view of the lid of Figure 1A along the line 1C-1C of Figure 1A, according to some embodiments;
Figure 1D is an upper perspective view of the lid of Figure 1A in a closed position, according to some embodiments;
Figure 2A is an upper perspective view of the lid of Figure 1A coupled to an example blood collection tube, illustrating an example closure of the lid in an open position, according to some embodiments;
Figure 2B is a cross-sectional view of the lid and the blood collection tube of Figure 2A along the line 2B-2B of Figure 2A, according to some embodiments;
Figure 2C is a cross-sectional view of the lid and the blood collection tube of Figure 2A along the line 2B-2B of Figure 2A, illustrating the closure in a closed position, according to some embodiments;
Figure 2D is a cross-sectional view of the lid and the blood collection tube of Figure 2A along the line 2D-2D of Figure 2A, according to some embodiments;
Figure 3A is an exploded view of an example blood collection apparatus, according to some embodiments;
Figure 3B is a cross-sectional view illustrating the lid of Figure 1A, an example connector, and an example catheter adapter coupled together, according to some embodiments; and
Figure 4 is an upper perspective view of the lid of Figure 1A having a second annular flange, according to some embodiments.

### DESCRIPTION OF EMBODIMENTS

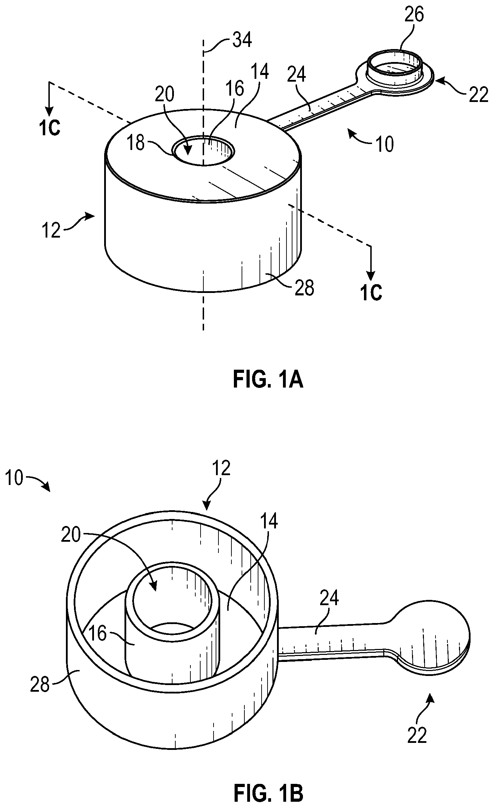

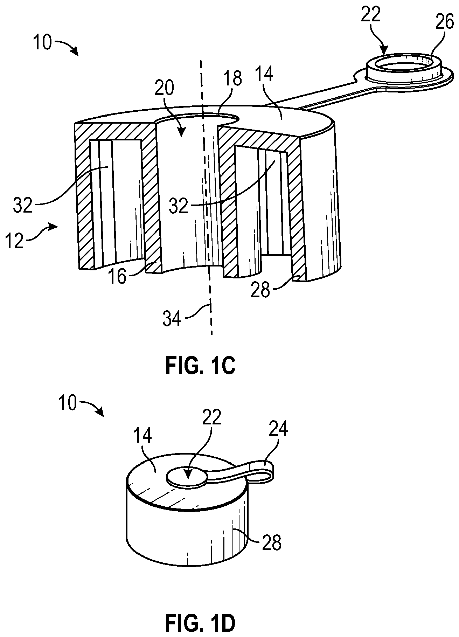

Referring now to Figures 1A-1D, in some embodiments, a lid 10 for a blood collection tube may include a body 12, which may include a cover portion 14 and an inner wall 16 extending from the cover portion 14. In some embodiments, the inner wall 16 may be aligned with an aperture 18 extending through the cover portion 14. In some embodiments, the inner wall 16 and the aperture 18 may form a channel 20, which may be configured to receive a male luer slip fitting of a connector. In some embodiments, the inner wall 16 may be tapered to receive the male luer slip fitting of the connector. In some embodiments, the inner wall 16 may not be tapered.

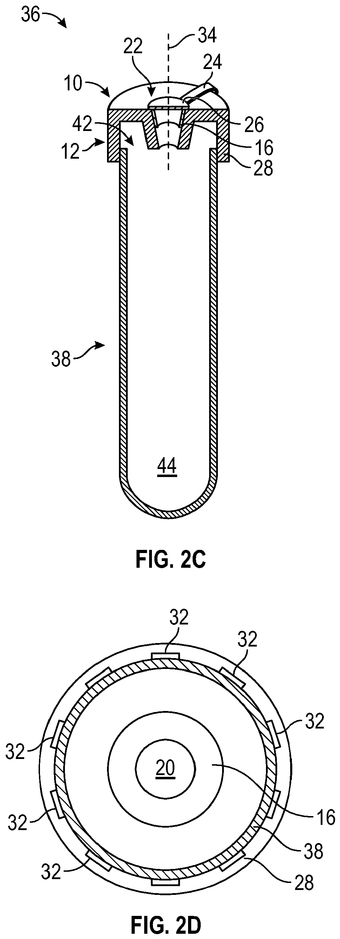

In some embodiments, the lid 10 may include a closure 22 and/or a tether 24 connecting the body 12 to the closure 22. In some embodiments, the closure 22 may include an annular flange 26, which may fit inside and contact a circumference of the channel 20 when the closure 22 is in a closed position, as illustrated, for example, in Figure 2D. In some embodiments, the annular flange 26 may include an outer circumference less than a circumference of the channel 20. In some embodiments, the annular flange 26 may include an outer circumference larger than a circumference of the channel 20 or approximately a same size as the circumference of the channel 20 such that the annular flange 26 is secured within the channel 20 in an interference fit.

In some embodiments, the body 12 of the lid 10 may include a generally cylindrical outer wall 28. In some embodiments, the tether 24 may extend from the outer wall 28 and/or the cover portion 14. In some embodiments, an outer surface of the cover portion 14 may be generally planar. In some embodiments, an inner surface of the cover portion disposed between the inner wall 16 and the outer wall 28 may be generally planar.

As illustrated in Figure 1C, in some embodiments, the inner surface of the outer wall 28 may include one or more grooves 32, which may extend generally parallel to a central axis 34 of the lid 10. In some embodiments, the grooves 32 may act as vents and have dimensions such that the grooves 32 are permeable to air but not blood.

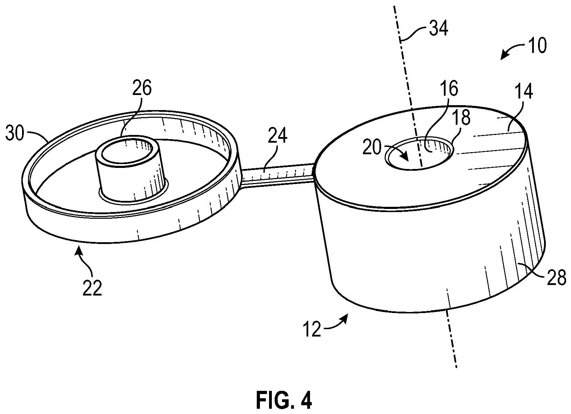

Referring now to Figure 4, in some embodiments, the closure 22 of the lid 10 may include another annular flange 30, which may contact the outer circumference of the outer wall 28 when the closure 22 is in a closed position. In some embodiments, the other annular flange 30 may include an inner circumference greater than the outer circumference of the outer wall 28. In some embodiments, the other annular flange 30 may include an inner circumference less than or approximately equal to the outer circumference of the outer wall.

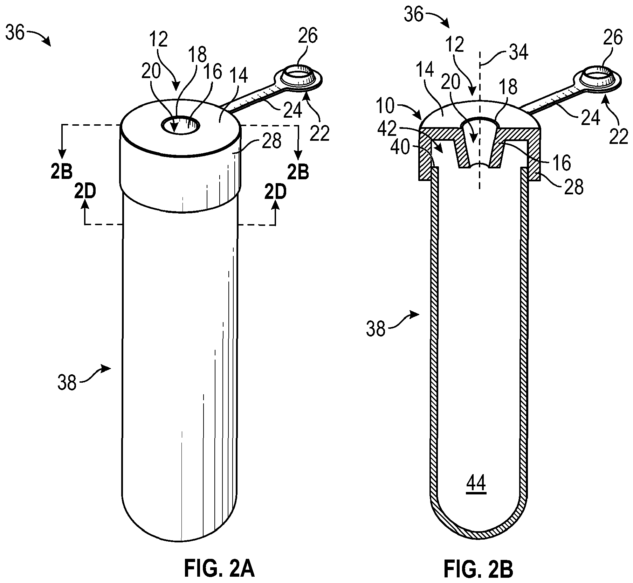

Referring now to Figures 2A-2D, in some embodiments, a blood collection apparatus 36 may include the lid 10 and/or a blood collection tube 38. In some embodiments, the blood collection tube 38 may be coupled to the lid 10. In some embodiments, the blood collection tube 38 may include a rim 40 forming an opening 42 into a cavity 44 of the blood collection tube 38. In some embodiments, the rim 40 may be annular. In some embodiments, the inner wall 16 of the lid 10 may extend a same distance as the outer wall 28, as illustrated in Figure 1C, for example, or a different distance, as illustrated in Figure 2B-2C, for example.

In some embodiments, the inner wall 16 of the body 12 may extend through the opening 42. In some embodiments, the rim 40 may contact the inner surface of the outer wall 28 of the body 12. Figure 2D illustrates the grooves 32, which may be generally parallel to the central axis of the lid, according to some embodiments. In some embodiments, air may pass between an exterior of the blood collection apparatus 36 and the cavity 44 of the blood collection tube 38 via the grooves 32, but the grooves 32 may be impermeable to blood to prevent leakage of blood from the cavity 44.

Figures 2C illustrates the lid 10 in a closed position, according to some embodiments. In some embodiments, the annular flange 26 may fit inside and contact a circumference of the channel 20 when the lid 10 is in the closed position. For example, the annular flange 26 may fit inside and contact a circumference of the aperture 18 and/or a circumference of the inner wall 16.

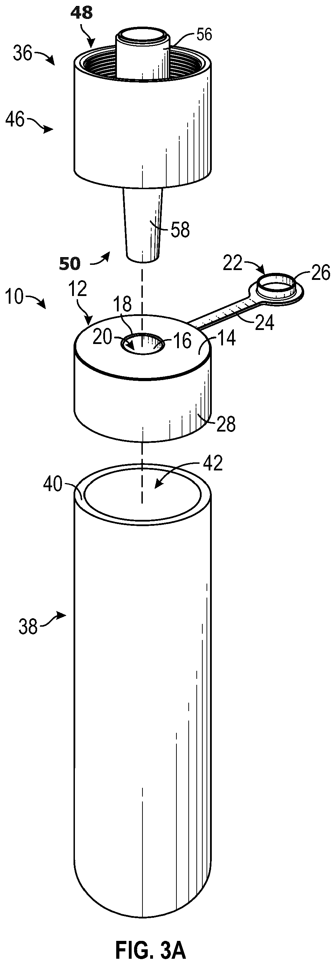

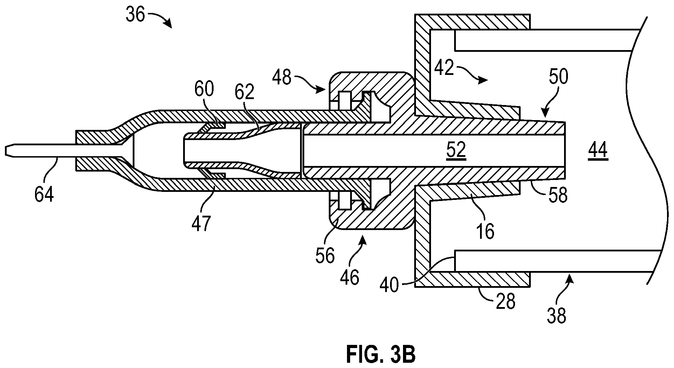

Referring now to Figures 3A-3B, in some embodiments, the blood collection apparatus 36 may include the connector 46 and/or a catheter adapter 47. In some embodiments, the connector 46 may include a distal end 48, a proximal end 50, and a lumen 52 extending there between. In some embodiments, the distal end 48 may include a male luer lock fitting 56 configured to couple to a proximal end of the catheter adapter 47, as illustrated, for example, in Figure 3B. In some embodiments, the proximal end of the catheter adapter 47 may include a female luer fitting. In some embodiments, the proximal end 50 may include a male luer slip fitting 58.

In some embodiments, the male luer lock fitting 56 may form a seal with the catheter adapter 47 or another medical device, preventing fluid leakage. In some embodiments, the male luer slip fitting 58 may seal the lid 10 when the male luer slip fitting 58 is inserted into the channel 20. In some embodiments, the male luer lock fitting 56 may include threading, which may form a sealed connection with the catheter adapter 47. In some embodiments, the male luer slip fitting 58 may act as a funnel, allowing blood to be channeled into the blood collection tube 38, while reducing or eliminating blood spill or splash.

In some embodiments, the catheter adapter 47 may include any catheter adapter known in the art. In some embodiments, the catheter adapter 47 may include a septum 60 and/or septum actuator 62. In other embodiments, the male luer slip fitting 56 may act as a septum actuator. In some embodiments, a catheter 64 may extend distally from a distal end of the catheter adapter 47. In some embodiments, the catheter 64 may include a peripheral intravenous catheter.

In some embodiments, the catheter 64 and the catheter adapter 47 may be used for a variety of infusion therapies. For example, the catheter 64 and the catheter adapter 47 may be used for infusing fluids, such as normal saline solution, various medicaments, and total parenteral nutrition, into a patient. In some embodiments, the catheter 64 and the catheter adapter 47 may also be used for withdrawing blood from the patient.

In some embodiments, the catheter 64 may include an over-the-needle peripheral intravenous catheter ("PIVC"), which may be mounted over an introducer needle having a sharp distal tip (not illustrated). In some embodiments, the catheter 64 and the introducer needle may be assembled so that the distal tip of the introducer needle extends beyond the distal tip of the catheter 64 with the bevel of the needle facing up away from skin of the patient. In some embodiments, the catheter 64 and introducer needle may be inserted at a shallow angle through the skin into vasculature of the patient.

In order to verify proper placement of the introducer needle and/or the catheter 64 in the vein, a user generally confirms that there is "flashback" of blood. Once placement of the needle has been confirmed, a user may temporarily occlude flow in the vein and remove the introducer needle, leaving the catheter 64 in place within the vein. In some embodiments, after the catheter 64 is placed within the vein and the introducer needle withdrawn, the lid 10 may be coupled to the catheter adapter 47 and blood may be collected in the blood collection tube 38.

## Claims

1. A blood collection apparatus (36), comprising:
a connector (46) comprising a distal end (48), a proximal end (50), and a lumen (52) extending there between, wherein the distal end comprises a male luer lock fitting (56) configured to couple to a proximal end of a catheter adapter (47), and wherein the proximal end (50) comprises a male luer slip fitting (58); and
a lid (10) for a blood collection tube (38),
wherein the lid comprises:
a body (12) having a generally cylindrical outer wall (28), a cover portion (14) and an inner wall (16) extending from the cover portion, wherein the inner wall is aligned with an aperture (18) extending through the cover portion, wherein the inner wall and the aperture form a channel (20) configured to receive the male luer slip fitting ;
a closure (22), wherein the closure comprises an annular flange (26) that fits inside and contacts a circumference of the channel when the closure is in a closed position, and wherein the closure further comprises another annular flange (30), wherein the other annular flange contacts the outer circumference of the outer wall when the closure is in the closed position; and
a tether (24) connecting the body to the closure.

2. The blood collection apparatus of claim 1, wherein the annular flange (26) comprises an outer circumference less than a circumference of the channel.

3. The blood collection apparatus of claim 1, wherein the annular flange (26) comprises an outer circumference larger than a circumference of the channel or approximately a same size as the circumference of the channel.

4. The blood collection apparatus of claim 1, wherein the other annular flange (30) comprises an inner circumference greater than the outer circumference of the outer wall.

5. The blood collection apparatus of claim 1, wherein the other annular flange (30) comprises an inner circumference less than or approximately equal to the outer circumference of the outer wall.

6. The blood collection apparatus of claim 1, wherein the inner wall is tapered to receive the male luer slip fitting.

7. The blood collection apparatus of claim 1, wherein an inner surface of the outer wall comprises a plurality of grooves (32) extending generally parallel to a central axis (34) of the lid.

8. The blood collection apparatus of claim 1, wherein an outer surface of the cover portion is generally planar.

9. The blood collection apparatus of claim 1, wherein an inner surface of the cover portion disposed between the inner wall and the outer wall is generally planar.

10. The blood collection apparatus of claim 1, further comprising the blood collection tube coupled to the lid, wherein the blood collection tube comprises a rim (40) forming an opening (42) into a cavity (44) of the blood collection tube, wherein the inner wall extends through the opening, wherein the rim contacts an inner surface of the outer wall of the body.

11. The blood collection apparatus of claim 10, wherein the inner surface of the outer wall comprises a plurality of grooves extending generally parallel to a central axis of the lid, wherein the plurality of grooves are permeable to air but not blood.

12. A blood collection apparatus (36), comprising:
a lid (10) for a blood collection tube (38),
wherein the lid comprises:
a body (12) having a generally cylindrical outer wall (28), a cover portion (14) and an inner wall (16) extending from the cover portion, the inner wall aligned with an aperture (18) extending through the cover portion, the inner wall and the aperture forming a channel (20);
a closure (22); and
a tether (24) connecting the body to the closure,
wherein the closure comprises an annular flange (26) that fits inside and contacts a circumference of the channel when the closure is in a closed position, and the closure further comprises another annular flange (30) that contacts an outer circumference of the outer wall when closure is in the closed position.

13. The blood collection apparatus of claim 12, wherein the inner wall is tapered.

14. The blood collection apparatus of claim 12, wherein an inner surface of the outer wall comprises a plurality of grooves (32) extending generally parallel to a central axis (34) of the lid.

15. The blood collection apparatus of claim 12, further comprising the blood collection tube (38) coupled to the lid, wherein the blood collection tube comprises a rim (40) forming an opening (42) into a cavity of the blood collection tube (44), wherein the inner wall extends through the opening, wherein the rim contacts an inner surface of the outer wall of the body.

16. The blood collection apparatus of claim 15, wherein the inner surface of the outer wall comprises a plurality of grooves (32) extending generally parallel to a central axis (34) of the lid, wherein the plurality of grooves are permeable to air but not blood.

17. The blood collection apparatus of claim 12, further comprising a connector (46) comprising a distal end (48), a proximal end (50), and a lumen (52) extending there between, wherein the distal end (48) comprises a male luer lock fitting (56) configured to couple to a proximal end of a catheter adapter (47), wherein the proximal end (50) comprises a male luer slipfitting (58), wherein the lid is coupled to the connector.

## Patentansprüche

1. Blutentnahmevorrichtung (36), die aufweist:
einen Verbinder (46), der ein distales Ende (48), ein proximales Ende (50) und ein sich dazwischen erstreckendes Lumen (52) aufweist, wobei das distale Ende einen männlichen Luer-Lock-Anschluss (56) aufweist, der dazu ausgebildet ist, sich mit einem proximalen Ende eines Katheteradapters (47) zu koppeln, und wobei das proximale Ende (50) einen männlichen Luer-Slip-Anschluss (58) aufweist; und
einen Deckel (10) für ein Blutentnahmeröhrchen (38), wobei der Deckel aufweist:
einen Körper (12) mit einer im Allgemeinen zylindrischen Außenwand (28), einem Abdeckabschnitt (14) und einer sich von dem Abdeckabschnitt erstreckenden Innenwand (16), wobei die Innenwand mit einer sich durch den Abdeckabschnitt erstreckenden Apertur (18) ausgerichtet ist, wobei die Innenwand und die Apertur einen Kanal (20) bilden, der dazu ausgebildet ist, den männlichen Luer-Slip-Anschluss aufzunehmen;
einen Verschluss (22), wobei der Verschluss einen ringförmigen Flansch (26) aufweist, der ins Innere passt und einen Umfang des Kanals berührt, wenn der Verschluss in einer geschlossenen Position ist, und wobei der Verschluss ferner einen anderen ringförmigen Flansch (30) aufweist, wobei der andere ringförmige Flansch den Außenumfang der Außenwand berührt, wenn der Verschluss in der geschlossenen Position ist; und
einen Haltegurt (24), der den Körper mit dem Verschluss verbindet.

2. Blutentnahmevorrichtung nach Anspruch 1, wobei der ringförmige Flansch (26) einen Außenumfang aufweist, der geringer als ein Umfang des Kanals ist.

3. Blutentnahmevorrichtung nach Anspruch 1, wobei der ringförmige Flansch (26) einen Außenumfang aufweist, der größer als ein Umfang des Kanals ist oder ungefähr die gleiche Größe wie der Umfang des Kanals hat.

4. Blutentnahmevorrichtung nach Anspruch 1, wobei der andere ringförmige Flansch (30) einen Innenumfang aufweist, der größer als der Außenumfang der Außenwand ist.

5. Blutentnahmevorrichtung nach Anspruch 1, wobei der andere ringförmige Flansch (30) einen Innenumfang aufweist, der kleiner als der oder ungefähr gleich dem Außenumfang der Außenwand ist.

6. Blutentnahmevorrichtung nach Anspruch 1, wobei die Innenwand verjüngt ist, um den männlichen Luer-Slip-Anschluss aufzunehmen.

7. Blutentnahmevorrichtung nach Anspruch 1, wobei eine Innenfläche der Außenwand eine Vielzahl von Nuten (32) aufweist, die sich im Allgemeinen parallel zu einer Mittelachse (34) des Deckels erstrecken.

8. Blutentnahmevorrichtung nach Anspruch 1, wobei eine Außenfläche des Abdeckabschnitts im Allgemeinen planar ist.

9. Blutentnahmevorrichtung nach Anspruch 1, wobei eine Innenfläche des Abdeckabschnitts, die zwischen der Innenwand und der Außenwand angeordnet ist, im Allgemeinen planar ist.

10. Blutentnahmevorrichtung nach Anspruch 1, die ferner das mit dem Deckel gekoppelte Blutentnahmeröhrchen aufweist, wobei das Blutentnahmeröhrchen einen Rand (40) aufweist, der eine Öffnung (42) in einen Hohlraum (44) des Blutentnahmeröhrchens bildet, wobei sich die Innenwand durch die Öffnung hindurch erstreckt, wobei der Rand eine Innenfläche der Außenwand des Körpers berührt.

11. Blutentnahmevorrichtung nach Anspruch 10, wobei die Innenfläche der Außenwand eine Vielzahl von Nuten aufweist, die sich im Allgemeinen parallel zu einer Mittelachse des Deckels erstrecken, wobei die Vielzahl von Nuten für Luft jedoch nicht für Blut durchlässig sind.

12. Blutentnahmevorrichtung (36), die aufweist:
einen Deckel (10) für ein Blutentnahmeröhrchen (38), wobei der Deckel aufweist:
einen Körper (12) mit einer im Allgemeinen zylindrischen Außenwand (28), einem Abdeckabschnitt (14) und einer sich von dem Abdeckabschnitt erstreckenden Innenwand (16), wobei die Innenwand mit einer sich durch den Abdeckabschnitt erstreckenden Apertur (18) ausgerichtet ist, wobei die Innenwand und die Apertur einen Kanal (20) bilden;
einen Verschluss (22); und
einen Haltegurt (24), der den Körper mit dem Verschluss verbindet,
wobei der Verschluss einen ringförmigen Flansch (26) aufweist, der ins Innere passt und einen Umfang des Kanals berührt, wenn der Verschluss in einer geschlossenen Position ist, und wobei der Verschluss ferner einen anderen ringförmigen Flansch (30) aufweist, der einen Außenumfang der Außenwand berührt, wenn der Verschluss in der geschlossenen Position ist.

13. Blutentnahmevorrichtung nach Anspruch 12, wobei die Innenwand verjüngt ist.

14. Blutentnahmevorrichtung nach Anspruch 12, wobei eine Innenfläche der Außenwand eine Vielzahl von Nuten (32) aufweist, die sich im Allgemeinen parallel zu einer Mittelachse (34) des Deckels erstrecken.

15. Blutentnahmevorrichtung nach Anspruch 12, die ferner das mit dem Deckel gekoppelte Blutentnahmeröhrchen (38) aufweist, wobei das Blutentnahmeröhrchen einen Rand (40) aufweist, der eine Öffnung (42) in einen Hohlraum (44) des Blutentnahmeröhrchens bildet, wobei sich die Innenwand durch die Öffnung hindurch erstreckt, wobei der Rand eine Innenfläche der Außenwand des Körpers berührt.

16. Blutentnahmevorrichtung nach Anspruch 15, wobei die Innenfläche der Außenwand eine Vielzahl von Nuten (32) aufweist, die sich im Allgemeinen parallel zu einer Mittelachse (34) des Deckels erstrecken, wobei die Vielzahl von Nuten für Luft jedoch nicht für Blut durchlässig sind.

17. Blutentnahmevorrichtung nach Anspruch 12, die ferner einen Verbinder (46) aufweist, der ein distales Ende (48), ein proximales Ende (50) und ein sich dazwischen erstreckendes Lumen (52) aufweist, wobei das distale Ende (48) einen männlichen Luer-Lock-Anschluss (56) aufweist, der dazu ausgebildet ist, sich mit einem proximalen Ende eines Katheteradapters (47) zu koppeln, wobei das proximale Ende (50) einen männlichen Luer-Slip-Anschluss (58) aufweist, wobei der Deckel mit dem Verbinder gekoppelt ist.

## Revendications

1. Appareil de collecte de sang (36), comprenant :
un connecteur (46) comprenant une extrémité distale (48), une extrémité proximale (50) et une lumière (52) s'étendant entre elles, dans lequel l'extrémité distale comprend un raccord luer mâle de verrouillage (56) conçu pour s'accoupler à une extrémité proximale d'un adaptateur de cathéter (47), et dans lequel l'extrémité proximale (50) comprend un raccord luer mâle coulissant (58) ; et
un couvercle (10) pour un tube de collecte de sang (38),
le couvercle comprenant :
un corps (12) ayant une paroi externe généralement cylindrique (28), une partie de couverture (14) et une paroi interne (16) s'étendant à partir de la partie de couverture, dans lequel la paroi interne est alignée avec l'orifice (18) s'étendant à travers la partie de couverture, dans lequel la paroi interne et l'orifice forment un canal (20) conçu pour recevoir le raccord luer mâle coulissant ;
une fermeture (22), la fermeture comprenant un rebord annulaire (26) qui s'ajuste à l'intérieur de, et vient en contact avec, une circonférence du canal lorsque la fermeture est dans une position fermée, et dans lequel la fermeture comprend en outre un autre rebord annulaire (30), dans lequel l'autre rebord annulaire vient en contact avec la circonférence externe de la paroi externe lorsque la fermeture est dans la position fermée ; et
une attache (24) reliant le corps à la fermeture.

2. Appareil de collecte de sang selon la revendication 1, dans lequel le rebord annulaire (26) comprend une circonférence externe inférieure à une circonférence du canal.

3. Appareil de collecte de sang selon la revendication 1, dans lequel le rebord annulaire (26) comprend une circonférence externe plus grande qu'une circonférence du canal ou approximativement de la même taille que la circonférence du canal.

4. Appareil de collecte de sang selon la revendication 1, dans lequel l'autre rebord annulaire (30) comprend une circonférence interne supérieure à la circonférence externe de la paroi externe.

5. Appareil de collecte de sang selon la revendication 1, dans lequel l'autre rebord annulaire (30) comprend une circonférence interne inférieure ou approximativement égale à la circonférence externe de la paroi externe.

6. Appareil de collecte de sang selon la revendication 1, dans lequel la paroi interne est effilée pour recevoir le raccord luer mâle coulissant.

7. Appareil de collecte de sang selon la revendication 1, dans lequel une surface interne de la paroi externe comprend une pluralité de rainures (32) s'étendant généralement parallèles à un axe central (34) du couvercle.

8. Appareil de collecte de sang selon la revendication 1, dans lequel une surface externe de la partie de couverture est généralement plane.

9. Appareil de collecte de sang selon la revendication 1, dans lequel une surface interne de la partie de couverture disposée entre la paroi interne et la paroi externe est généralement plane.

10. Appareil de collecte de sang selon la revendication 1, comprenant en outre le tube de collecte de sang couplé au couvercle, dans lequel le tube de collecte de sang comprend une couronne (40) formant une ouverture (42) dans une cavité (44) du tube de collecte de sang, dans lequel la paroi interne s'étend à travers l'ouverture, dans lequel la couronne vient en contact avec une surface interne de la paroi externe du corps.

11. Appareil de collecte de sang selon la revendication 10, dans lequel la surface interne de la paroi externe comprend une pluralité de rainures s'étendant généralement parallèles à un axe central du couvercle, dans lequel la pluralité de rainures sont perméables à l'air mais pas au sang.

12. Appareil de collecte de sang (36), comprenant :
un couvercle (10) pour un tube de collecte de sang (38),
le couvercle comprenant :
un corps (12) ayant une paroi externe généralement cylindrique (28), une partie de couverture (14) et une paroi interne (16) s'étendant à partir de la partie de couverture, la paroi interne alignée avec un orifice (18) s'étendant à travers la partie de couverture, la paroi interne et l'orifice formant un canal (20) ;
une fermeture (22) ; et
une attache (24) reliant le corps à la fermeture,
dans lequel la fermeture comprend un rebord annulaire (26) qui s'ajuste à l'intérieur de, et vient en contact avec, une circonférence du canal lorsque la fermeture est dans une position fermée, et la fermeture comprend en outre un autre rebord annulaire (30) qui vient en contact avec une circonférence externe de la paroi externe lorsque la fermeture est dans la position fermée.

13. Appareil de collecte de sang selon la revendication 12, dans lequel la paroi interne est effilée.

14. Appareil de collecte de sang selon la revendication 12, dans lequel une surface interne de la paroi externe comprend une pluralité de rainures (32) s'étendant généralement parallèles à un axe central (34) du couvercle.

15. Appareil de collecte de sang selon la revendication 12, comprenant en outre le tube de collecte de sang (38) couplé au couvercle, dans lequel le tube de collecte de sang comprend une couronne (40) formant une ouverture (42) dans une cavité du tube de collecte de sang (44), dans lequel la paroi interne s'étend à travers l'ouverture, dans lequel la couronne vient en contact avec une surface interne de la paroi externe du corps.

16. Appareil de collecte de sang selon la revendication 15, dans lequel la surface interne de la paroi externe comprend une pluralité de rainures (32) s'étendant généralement parallèles à un axe central (34) du couvercle, dans lequel la pluralité de rainures sont perméables à l'air mais pas au sang.

17. Appareil de collecte de sang selon la revendication 12, comprenant en outre un connecteur (46) comprenant une extrémité distale (48), une extrémité proximale (50) et une lumière (52) s'étendant entre elles, dans lequel l'extrémité distale (48) comprend un raccord luer mâle de verrouillage (56) conçu pour s'accoupler à une extrémité proximale d'un adaptateur de cathéter (47), dans lequel l'extrémité proximale (50) comprend un raccord luer mâle coulissant (58), dans lequel le couvercle est couplé au connecteur.
